# EUROPÄISCHE PATENTSCHRIFT

(11) **EP 3 079 993 B1**
(45) Veröffentlichungstag und Bekanntmachung des Hinweises auf die Patenterteilung: **19.08.2020**
(21) Anmeldenummer: 14805560.1
(22) Anmeldetag: 27.11.2014
(51) Int. Cl.: B65B 55/08, A61L 2/00, B65B 55/10

(54) **VERFAHREN ZUM ENTKEIMEN VON PACKMITTELN**
METHOD FOR STERILIZING PACKAGING
PROCÉDÉ D'ASEPTISATION DE MOYENS D'EMBALLAGE

(30) Priorität: 10.12.2013 DE 102013113784
(43) Veröffentlichungstag der Anmeldung: 19.10.2016
(73) Patentinhaber: KHS GmbH, 44143 Dortmund (DE)
(72) Erfinder: CLÜSSERATH, Ludwig, 55543 Bad Kreuznach (DE)
(86) Internationale Anmeldenummer: PCT/EP2014/075752
(87) Internationale Veröffentlichungsnummer: WO 2015/086329

(56) Entgegenhaltungen:
- DE-A1-102010 044 244
- US-A- 2 380 984
- US-A1- 2009 134 338

## Beschreibung

Die Erfindung bezieht sich auf ein Verfahren gemäß Oberbegriff Patentanspruch 1.

Speziell im Lebensmittel- und/oder Getränkebereich ist es vielfach erforderlich produkt- oder füllgutführende Oberflächen, d.h. insbesondere die mit einem Produkt oder Füllgut in Berührung kommenden Flächen oder Oberflächen von Packmitteln und Packmittelverschlüssen, aber auch Maschinenflächen, die mit einem Produkt oder Füllgut und/oder mit einem in die Packmittel beim Füllen eingeleiteten Behandlungsmedium oder Betriebsmittel in Berührung kommen, zum Abtöten von Keimen, insbesondere auch zum Abtöten von Produkt schädigenden Keimen, wie Pilze, Bakterien, Viren usw. zu entkeimen oder zu sterilisieren, beispielsweise durch Behandlung mit einem geeigneten Desinfektionsmittel.

Bekannt ist dabei die Verwendung von Chlordioxid (ClO2) enthaltenden wässrigen Lösungen als Desinfektionsmittel zum Entkeimen oder Sterilisieren von Packmitteln (DE 20 2012 011 289 U1) sowie auch die Verwendung von Chlorverbindungen in wässrigen, CO2-enthaltenden Lösungen zur Entkeimung nicht nur von Packmitteln, wie KEGs, Flaschen und dergleichen, sondern auch von Anlagen- und Maschinenkomponenten im Nahrungs- und Lebensmittelbereich, wie Tanks, Rohrleitungen usw. (DE 196 44 251 A1). Bekannt sind dabei speziell auch Verfahren zum Entkeimen von Packmittel in Form von Flaschen unter Verwendung von Chlordioxid als Desinfektionsmittel bei gleichzeitiger Beaufschlagung der jeweiligen Oberfläche mit einer UV-Strahlung zur Erhöhung der Wirkung von Chlordioxid (CN 102826261 A1).

Daneben ist aus der US 2,380,984 A eine Vorrichtung sowie ein Verfahren zum Konservieren von Produkten, insbesondere von flüssigen oder zähen Lebens-/ Nahrungsmitteln, bekannt. Hierbei werden die zu füllenden Behälter zunächst durch eine erste Sterilisationseinheit und eine zweite Sterilisationseinheit geführt, in welchen die Behälter jeweils mittels Frisch- bzw. Wasserdampf erhitzt werden. Sofern die Behälter im Anschluss an die Sterilisationseinheiten eine Kühleinheit durchlaufen, müssen diese oftmals erneut sterilisiert werden, und zwar in dem diese durch eine mit einer chlorhaltigen Flüssigkeit gefüllten Behandlungskammer geführt werden. Im Anschluss daran werden die Behälter durch einen sterilen Arbeitsraum geführt, in welchem unterschiedliche Arbeitsschritte erfolgen können wie z.B. das Einbringen einer Einfüllöffnung in den Deckel des Behälters oder das Befüllen des Behälters. Zur Erzeugung einer sterilen Umgebung in dem Arbeitsraum ist der Arbeitsraum mit einem Schutzgas wie z.B. Stickstoff oder Kohlendioxid gefüllt, wobei das Schutzgas in dem Arbeitsraum unter einem Druck steht, welcher höher ist als der Druck außerhalb des Arbeitsraums.

Bekannt sind weiterhin Verfahren zur Entkeimung von Oberflächen von Gegenständen, wobei die jeweilige Oberfläche bei Anwesenheit von UV-Strahlung mit Wasserstoffperoxid oder mit Ozon behandelt wird (EP 0 341 069 A2).

Bekannt ist ferner zum Entkeimen von Gegenständen, insbesondere von Medizinprodukten diese Gegenstände mit Elektronenstrahlen zu behandeln, und zwar in einer Behandlungskammer, die hierbei eine Atmosphäre aus Stickstoff, Sauerstoff, Kohlendioxid, gasförmiges Wasserstoffperoxid und/oder Argon enthält (WO 2013/0954539 A1).

Bekannt ist weiterhin zum Entkeimen von Behältern diese in zwei zeitlich auf einander folgenden Verfahrensschritten jeweils mit einer wässrigen Lösung von Chlordioxid zu behandeln, wobei die Konzentration des Chlordioxids in der Lösung des ersten, zeitlich vorausgehenden Behandlungsschritts höher ist als die Chlordioxid-Konzentration in dem folgenden Behandlungsschritt (DE 20 2012 011 289 U1).

Bekannt ist schließlich das Entkeimen von Gegenständen in Form von versandten Briefumschlägen, Paketen und Verpackungen unter Verwendung einer antimikrobiellen Flüssigkeit, die beispielsweise Chlordioxid enthält. Das Entkeimen erfolgt dabei durch Beaufschlagung der Gegenstände mit Mikrowellenenergie und mit UV-Strahlung (US 7 067 089 B2).

Aufgabe der Erfindung ist es, ein besonders wirksames Verfahren zum Entkeimen oder Sterilisieren von Innenflächen von Behältern aufzuzeigen. Zur Lösung dieser Aufgabe ist ein Verfahren entsprechend dem Patentanspruch 1 ausgebildet.

"Packmittel" sind im Sinne der Erfindung Verpackungen oder Behältnisse, die im Lebensmittelbereich und dabei speziell auch im Getränkebereich üblicherweise verwendet werden, und zwar insbesondere Behälter, wie z. B. Flaschen, Dosen, KEGs, aus Glas, Kunststoff, Metall, aber auch Weichverpackungen, beispielsweise solche hergestellt aus Karton und/oder Kunststofffolie und/oder Metallfolie. "Produkt- oder füllgut-kritische Oberflächen" sind im Sinne der Erfindung zunächst "produkt- oder füllgut-führende Oberflächen", d.h. solche Oberflächen, die mit einem Produkt oder Füllgut in Berührung kommen oder in Berührung stehen, insbesondere Innenflächen von Packmitteln und/oder von Packmittel-Verschlüssen, aber auch in Maschinen und Anlagen gebildete Oberflächen, mit denen das jeweilige Produkt oder Füllgut zumindest zeitlich begrenzt in Berührung kommt oder steht, wie z.B. die Innenflächen von Produkt- oder Füllgut-Tanks, Produkt- oder Füllgut-Leitungen, Füllelemente usw. "Produkt- oder füllgut-kritische Oberflächen" sind im Sinne der Erfindung aber auch solche Oberflächen insbesondere in Anlagen und Maschinen, die zumindest zeitlich begrenzt mit einem Medium in Berührung kommen oder stehen, welches beim Füllen der Packmittel als Betriebsmittel verwendet in die Packmittel eingeleitet wird, beispielsweise als Spülgas oder aber als Spanngas beim Druckfüllen von Flaschen oder dergleichen Behältern.

Unter "Druckfüllen" ist dabei im Sinne der Erfindung allgemein ein Füllverfahren zu verstehen, bei dem der jeweils zu füllende Behälter vor dem eigentlichen Füllen mit einem unter Druck stehenden Spanngas (Inertgas bzw. CO2-Gas) vorgespannt wird, welches dann während des Füllens von dem dem Behälter zufließenden Füllgut zunehmend als Rückgas aus den Behälterinnenraum verdrängt wird.

Die Besonderheit des erfindungsgemäßen Verfahrens besteht darin, dass die Behandlung der jeweiligen zu entkeimenden Oberfläche mit dem Desinfektionsmittel durch Beaufschlagung mit einem keimschädigenden Energieeintrag und/oder mit einem keimschädigenden Behandlungsmedium unterstützt wird. Hierbei wird mit diesem keimschädigenden Energieeintrag und/oder mit diesem keimschädigenden Behandlungsmedium die Zellstruktur, insbesondere die äußere Hülle oder Membrane der Keime aufgebrochen, wodurch ein verbessertes und schnelleres Eindringen des das Desinfektionsmittels in die Keime erreicht und die Qualität der Entkeimung (Entkeimungsrate) bei reduzierter Behandlungsdauer verbessert wird.

Der Energieeintrag erfolgt durch Licht, vorzugsweise durch UV-Licht oder UV-Strahlung, durch Lichtblitze, durch Ultraschall, durch elektromagnetische Strahlung und/oder durch Elektronenstrahlung.

Als keimschädigendes Behandlungsmedium eignen sich beispielsweise Chlorverbindungen, z.B. Chlordioxid (ClO2) oder ein diese Chlorverbindungen als wirksame Komponente enthaltendes Medium, welches über Düsen auf die Innenflächen von Behältern aufgebracht wird.

Als Desinfektionsmittel oder als wirksame, d.h. keimtötende Komponente des Desinfektionsmittels kann eine Komponente verwendet werden, die umweltverträglich ist und insbesondere auch die Gefahr von Verletzungen des Bedienungspersonals vermeidet, beispielsweise Kohlendioxid (CO2), und/oder das Desinfektionsmittel kann die keimtötende Komponente, beispielsweise eine Chlorverbindung enthalten, vorzugsweise Chlordioxid (ClO2) in einer nur reduzierten Dosierung, z.B. in einer im Vergleich zu dem keimschädigenden Behandlungsmedium reduzierten Dosierung.

Das Desinfektionsmittel sowie auch das keimschädigende Behandlungsmedium kommen bei dem erfindungsgemäßen Verfahren entweder in gas- und/oder dampfförmiger Form oder aber in flüssiger Form, beispielsweise als wässrige Lösung zum Einsatz, welches über Düsen auf die Innenflächen von Behältern aufgebracht wird.

Der Ausdruck "im Wesentlichen" bzw. "etwa" bzw. "ca." bedeutet im Sinne der Erfindung Abweichungen vom jeweils exakten Wert um +/- 10%, bevorzugt um +/-5% und/oder Abweichungen in Form von für die Funktion unbedeutenden Änderungen.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an Ausführungsbeispielen näher erläutert. Es zeigen:
- Fig. 1: in schematischer Darstellung eine Abfolge verschiedener Verfahrensschritte zum Entkeimen oder Sterilisieren von Oberflächen, insbesondere von Produkt-oder Füllgut führenden Oberflächen bei einer Ausführungsform des erfindungsgemäßen Verfahrens;
- Fig. 2: in den Positionen a - c nochmals in vergrößerter Darstellung eine Oberfläche bei der Behandlung mit dem Verfahren der Figur 1;
- Fig. 3: eine Darstellung ähnlich der Figur 1 bei einer weiteren Ausführungsform des erfindungsgemäßen Verfahrens.

In den Figuren ist 1 ein Gegenstand, der dort nur sehr schematisch und in Teildarstellung wiedergegeben ist und der bei seiner Verwendung mit wenigstens einer Oberfläche 2 mit einem Produkt oder Füllgut, beispielsweise Getränk in Berührung kommt und aus diesem Grund zumindest an dieser Oberfläche 2 entkeimt oder sterilisiert werden muss.

Der Gegenstand 1 ist beispielsweise ein mit einem Produkt oder Füllgut, z.B. Getränk zu füllender Behälter, ein Behälterverschluss oder aber ein Funktionselement einer Behälterbehandlungsmaschine, wobei in diesem Fall die Oberfläche 2 eine Maschinenfläche bildet, die mit dem Produkt oder Füllgut oder einem Betriebsmittel in Berührung steht oder kommt.

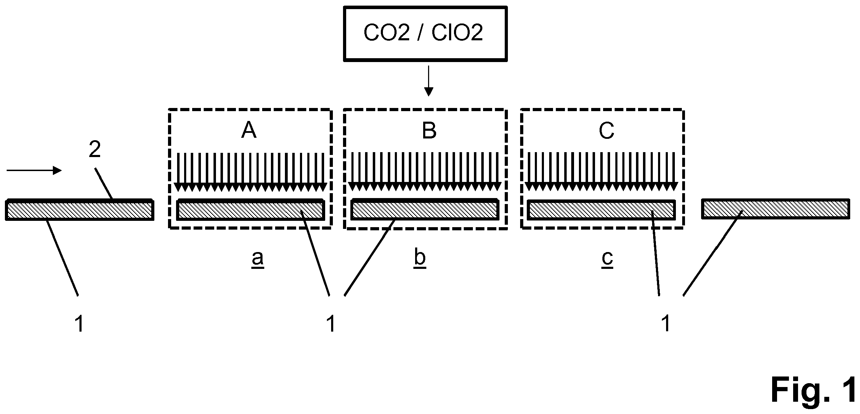

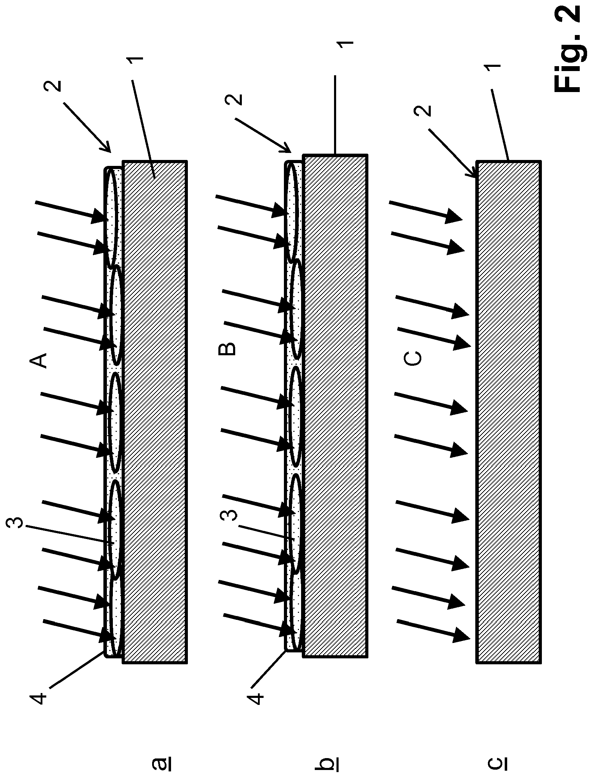

Zum Entkeimen oder Sterilisieren wird die Oberfläche 2 in einem ersten Verfahrensschritt entsprechend der Position a der Figuren 1 und 2 zunächst mit einem vorzugsweise aggressiven keimschädigenden Behandlungsmedium, beispielsweise mit Chlordioxid (ClO2) und/oder mit einem Energieeintrag beaufschlagt, und zwar in Form von Licht, Lichtblitzen, insbesondere UV-Licht, Ultraschall, elektromagnetischer Strahlung, Elektronenstrahlung usw., wie dies in der Position a mit den dortigen Pfeilen A angedeutet ist. Durch diese Behandlung wird ein Schädigen oder Aufbrechen der Keime oder deren Zellstruktur und dabei insbesondere der Zellwandung oder Membrane der an der Oberfläche 2 haftenden Keime 3 sowie auch einer diese Keime 3 enthaltenden Oberflächenschicht 4 erreicht. Das keimschädigende Behandlungsmedium wird beispielsweise gasförmig und/oder in wässriger Lösung, beispielsweise über Dosierdüsen auf die Oberfläche 2 aufgebracht.

Selbstverständlich kann die Behandlung sowohl durch Energieeintrag als auch durch Beaufschlagung der Oberfläche 2 mit dem keimschädigenden Behandlungsmedium erfolgen, und zwar zeitlich versetzt oder zeitgleich oder zeitlich überlappend.

Nach dem Schädigen oder Aufbrechen der Keime 3 erfolgt entsprechend der Position b der Figuren 1 und 2 in einem weiteren Verfahrensschritt die Behandlung der Oberfläche 2 mit dem Desinfektionsmittel. Im Gegensatz zu bekannten Verfahren zum Entkeimen und/oder Sterilisieren von Behältern, Behälterverschlüssen und/oder Maschinenoberflächen mit eine Desinfektionsmittel kann bei dem erfindungsgemäßen Verfahren ein besonders sanftes, umweit- und/oder maschinenschonendes Desinfektionsmittel eingesetzt werden, welches auch die Gefahr von Verletzungen des Bedienungspersonals vermeidet. Dies ist dadurch möglich, dass im Verfahrensschritt a die Keime 3 bzw. deren Zellstruktur oder Zellmembranen sowie auch die Schicht 4 bereits soweit geschädigt bzw. aufgebrochen wurden, dass im Verfahrensschritt b das sanfte Desinfektionsmittel zum sicheren Abtöten der vorgeschädigten Keime 3 ausreicht. Hierbei lässt sich eine Abtötungsrate erzielen, die bei den bisher üblichen Verfahren nur mit sehr intensiven Desinfektionsmitteln erreicht werden können.

Als Desinfektionsmittel eignet sich für den Verfahrensschritt b Kohlendioxid (CO2), dessen keimtötende Wirkung darauf beruht, dass in die Keime 3 eindringendes Kohlendioxid sich dort anreichert und zu einer von dem jeweiligen Keim 3 nicht mehr zu regulierenden Absenkung des pH-Wertes führt, und zwar mit der Folge, dass der betreffende Keim 3 unmittelbar abstirbt.

Als Desinfektionsmedium eignet sich für den Verfahrensschritt b auch Chlordioxid (ClO2), welches dann allerdings in einer gegenüber dem Verfahrensschritt a reduzierten Dosierung eingesetzt werden kann.

Das Desinfektionsmittel wird im Verfahrensschritt b in Gasform und/oder in wässriger Lösung auf die Oberfläche 2 aufgebracht, wie dies mit den Pfeilen B angedeutet ist. Wird als Desinfektionsmittel CO2 in wässriger Lösung verwendet, so enthält es das CO2 beispielsweise in einer Konzentration bis zu 2,5 gr. pro Liter Wasser und zwar bei normalen Umgebungs- bzw. Atmosphärendruck.

Durch die Schädigung der Keime 3 und durch das Aufbrechen der Oberflächenschicht 4 im Verfahrensschritt a erfolgt im Verfahrensschritt b eine zuverlässige Entkeimung und Sterilisation auch solcher Bereiche der Oberfläche 2, die im Verfahrensschritt a wegen "Verschattung" durch den Energieeintrag nicht oder nur ungenügend vorbehandelt wurden, sofern diese Bereiche flächenmäßig nur einen geringeren Teil, beispielsweise weniger als 10% der Gesamtfläche der Oberfläche 2 ausmachen.

In einem weiteren Verfahrensschritt entsprechend der Position c der Figuren 1 und 2 erfolgt dann bevorzugt noch ein Abspülen der Oberfläche 2 und damit ein vollständiges Entfernen der abgetöteten Keime 3 und der Oberflächenschicht 4 mit einem geeigneten Spülmedium, beispielsweise mit sterilem Wasser, wie dies mit den Pfeilen C angedeutet ist.

Vorstehend wurde davon ausgegangen, dass der Gegenstand 1 zum Entkeimen oder Sterilisieren nur an einer Oberfläche 2 behandelt wird. Es versteht sich, dass diese Behandlung an sämtlichen Oberflächen 2 des jeweiligen Gegenstandes 1 erfolgt, die steril oder keimfrei sein müssen. Die vorbeschriebenen Verfahrensschritte a - c werden dann an sämtlichen Oberflächen durchgeführt.

Vorstehend wurde davon ausgegangen, dass der Energieeintrag unmittelbar auf die zu entkeimende oder zu sterilisierende Oberfläche 2 erfolgt. Grundsätzlich besteht bei Gegenständen 1, die für die verwendete Energiestrahlung (Licht, UV-Licht, elektromagnetische Strahlung, Elektronenstrahlung) durchgängig sind, auch die Möglichkeit, den Energieeintrag an der zu entkeimenden oder zu sterilisierenden Oberfläche 2 zumindest zusätzlich unter Durchstrahlung des Gegenstandes 1 zu bewirken.

Handelt es sich bei den Gegenständen 1 um Behälter, so wird das vorstehend beschriebene Verfahren beispielsweise in einer Anlage oder Maschine zum Reinigen und/oder Sterilisieren der Behälter, beispielsweise in einem Rinser durchgeführt und das keimschädigende Behandlungsmedium sowie das Desinfektionsmedium werden über entsprechende Düsen insbesondere in die Behälter bzw. auf die zu entkeimenden oder zu sterilisierenden Innenflächen der Behälter aufgebracht. Dient das Verfahren zum Entkeimen und/oder Sterilisieren von Behälterverschlüssen, so wird dieses Verfahren beispielsweise in einer Anlage oder einer Anlagenkomponente durchgeführt, die eine Verschließmaschine mit den sterilisierten Behälterverschlüssen versorgt.

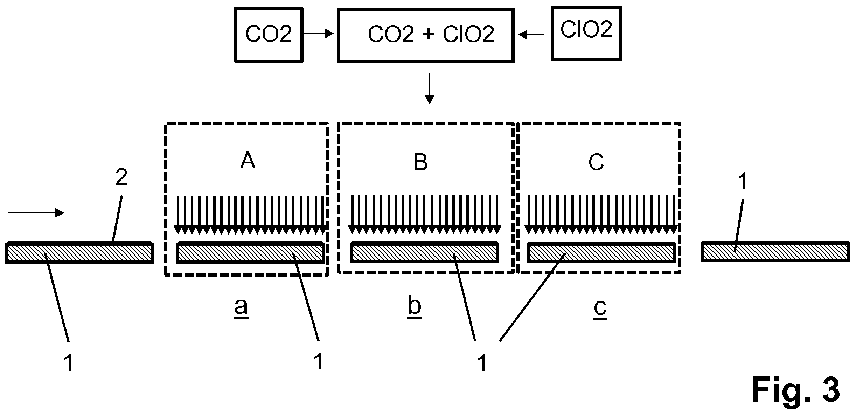

Die Figur 3 zeigt in den Positionen a - c die Verfahrensschritte eines weiteren Verfahrens gemäß der Erfindung, welches sich von dem Verfahren der Figur 1 lediglich dadurch unterscheidet, dass während der Desinfektion bzw. im Verfahrensschritt b ein Desinfektionsmittel verwendet wird, welches in Mischung wenigstens zwei Komponenten enthält, beispielsweise ClO2 versetzt mit CO2. Die beiden Komponenten des Desinfektionsmittels werden vorgemischt oder vordosiert in wässriger Lösung oder gasförmig bereit gestellt und auf die zu entkeimende oder zu sterilisierende Oberfläche 2 ausgebracht. Um zu vermeiden, dass es bereits bei einer Mischung oder Dosierung der beiden Komponenten unter atmosphärischem Druck zu einer Gasentbindung, d.h. zu einem Ausgasen von CO2 kommt, wird das Desinfektionsmittel bei der Dosierung und bis zum Ausbringen auf die Oberfläche 2 unter Druck gehalten, sodass die Gasentbindung erst beim Austritt des Desinfektionsmittels aus einer Behandlungsdüse, beispielsweise aus der Behandlungsdüse einer Behälterreinigungs- und/oder Desinfektionsanlage stattfindet und dadurch zur Erzielung einer hoher Entkeimungsrate bei reduzierter Behandlungsdauer die Wirksamkeit des Desinfektionsmittels voll erhalten bleibt.

Unabhängig davon, ob das von wenigstens zwei Komponenten gebildete Desinfektionsmittel gasförmig oder aber in flüssiger Form, beispielsweise als wässrige Lösung auf die Oberfläche 2 aufgebracht wird, ist es weiterhin auch möglich, die Komponenten des Desinfektionsmittels erst unmittelbar beim Ausbringen auf die Oberfläche 2 zu mischen, beispielsweise durch Verwendung von Mischdüsen oder Düsenanordnungen, die für jede Komponente wenigstens eine eigene Düse aufweisen. Sind die Gegenstände 1 Behälter, so sind die Mischdüsen oder Düsenanordnungen jeweils Bestandteil einer Behandlungsposition einer Maschine zum Reinigen und/oder Sterilisieren von Behältern, beispielsweise eines Rinsers.

Die beschriebenen Verfahrensschritte a - c werden beispielsweise jeweils an ein und derselben Behandlungsposition durchgeführt, wobei der Gegenstand 1 bis zum Abschluss des Verfahrens an der jeweiligen Behandlungsposition verbleibt.

Grundsätzlich besteht aber auch die Möglichkeit, dass die Verfahrensschritte a - c oder aber zumindest zwei dieser Verfahrensschritte an unterschiedlichen Behandlungspositionen durchgeführt werden, wobei der Gegenstand 1 dann von einer Behandlungsposition an die Behandlungsposition des nächsten Verfahrensschrittes weiterbewegt wird.

Vorstehend wurde auch der besseren Übersichtlichkeit halber davon ausgegangen, dass die Verfahrensschritte a - c jeweils zeitlich nacheinander ausgeführt werden. Bei dem erfindungsgemäßen Verfahren ist es aber auch möglich, dass die die Wirkung des Desinfektionsmittels unterstützende Behandlung mit dem keimschädigenden Behandlungsmedium und/oder mit dem keimschädigenden Energieeintrag (vorstehender Verfahrensschritt a) zeitgleich mit der Behandlung mit dem Desinfektionsmittel oder zumindest teilweise zeitlich überlappend mit der Behandlung mit dem Desinfektionsmittel erfolgt.

Die Erfindung wurde voranstehend an Ausführungsbeispielen beschrieben. Es versteht sich, dass zahlreiche Änderungen sowie Abwandlungen möglich sind, ohne dass dadurch der der Erfindung zugrundeliegende Erfindungsgedanke verlassen wird.

### Bezugszeichenliste

- 1: Gegenstand
- 2: zu behandelnde Oberfläche
- 3: Keim
- 4: Oberflächenschicht

## Patentansprüche

1. Verfahren zur Entkeimung von Innenflächen (2) von Behältern durch deren Behandlung mit einem Desinfektionsmittel, wobei die Innenflächen (2) mit einem keimschädigenden Energieeintrag in Form von UV-Licht und/oder in Form von Ultraschall und/oder mit einem keimschädigenden Behandlungsmedium behandelt wird, welches eine Chlorverbindung enthält und welches über Düsen auf die Innenflächen (2) aufgebracht wird, und das Desinfektionsmittel Kohlendioxid (CO2) ist und in flüssiger oder wässriger Form und/oder in Gas- und/oder Dampfform über Düsen auf die Innenflächen (2) aufgebracht wird, wobei durch die Behandlung der Innenflächen (2) mit dem keimschädigenden Energieeintrag und/oder mit dem keimschädigenden Behandlungsmedium die Zellstruktur, insbesondere die äußere Hülle oder Membrane von Keimen aufgebrochen wird, und wobei die Beaufschlagung der Innenflächen (2) mit dem keimschädigenden Energieeintrag und/oder mit dem keimschädigenden Behandlungsmedium zeitlich vorder Behandlung der Innenflächen (2) mit dem Desinfektionsmittel erfolgt.

2. Verfahren nach Anspruch 1, **dadurch gekennzeichnet, dass** das Desinfektionsmittel ebenfalls die Chlorverbindung enthält, und zwar in einer Dosierung, die kleiner ist als die Dosierung der Chlorverbindung in dem keimschädigenden Behandlungsmedium.

3. Verfahren nach Anspruch 1 oder 2, **dadurch gekennzeichnet, dass** das keimschädigende Behandlungsmedium in flüssiger oder wässriger Form und/oder in Gas- und/oder Dampfform auf die Innenflächen (2) aufgebracht wird.

4. Verfahren nach einem der vorhergehenden Ansprüche, **dadurch gekennzeichnet, dass** der Energieeintrag durch UV-Lichtblitze erfolgt.

5. Verfahren nach einem der vorhergehenden Ansprüche, **dadurch gekennzeichnet, dass** das keimschädigende Behandlungsmedium eine Chlorverbindung ist.

6. Verfahren nach einem der vorhergehenden Ansprüche, **dadurch gekennzeichnet, dass** das Kohlendioxid (CO2) und die Chlorverbindung enthaltende Desinfektionsmittel unter einem über dem Umgebungsdruck liegenden Druck bereitgestellt und erst bei einem Ausbringen auf die Innenflächen (2) auf Umgebungsdruck entspannt wird.

7. Verfahren nach einem der vorhergehenden Ansprüche, **dadurch gekennzeichnet, dass** die Chlorverbindung des Desinfektionsmittels und/oder des keimschädigenden Behandlungsmediums Chlordioxid (ClO2) ist.

8. Verfahren nach einem der vorhergehenden Ansprüche, **dadurch gekennzeichnet, dass** das Desinfektionsmittel und/oder das keimschädigende Behandlungsmedium in einem gas- und/oder dampfförmigen Zustand oder in einem flüssigen Zustand verwendet wird, beispielsweise in Form einer wässrigen Lösung.

## Claims

1. Method for sterilising interior surfaces (2) of containers by treating them with a disinfectant agent, wherein the interior surfaces (2) are treated with a sterilising application of energy in the form of UV light and/or in the form of ultrasonics and or with a disinfecting treatment medium which contains a chlorine compound, which is applied by means of nozzles onto the interior surfaces (2), the disinfectant agent is carbon dioxide (CO2) and is applied in fluid or aqueous form and/or in gaseous or vapour form by means of nozzles onto the interior surfaces (2), wherein, due to the treatment of the interior surfaces (2) by the sterilising energy application and/or with the disinfecting treatment medium, the cell structure of germs, in particular the outer sheaths or membranes, are broken open and wherein the treatment of the inner surfaces (2) with the disinfecting energy application and/or of the disinfecting treatment medium takes place before the treatment of the inner surfaces (2) with the disinfectant agent.

2. Method according to claim 1, **characterised in that** the disinfectant agent likewise contains the chlorine compound, and specifically in a dosing quantity which is smaller that the dosing quantity of the chlorine compound in the disinfectant treatment medium.

3. Method according to claim 1 or 2, **characterised in that** the disinfectant treatment medium is applied in fluid or aqueous form and/or in gaseous or vapour form onto the inner surfaces (2).

4. Method according to any one of the preceding claims, **characterised in that** the energy application takes place by flashes of UV light.

5. Method according to any one of the preceding claims, **characterised in that** the disinfectant treatment medium is a chlorine compound.

6. Method according to any one of the preceding claims, **characterised in that** the carbon dioxide (CO2) and the disinfectant agent containing the chlorine compound is applied under a pressure which is above ambient pressure and is only relaxed to ambient pressure after an application onto the inner surfaces (2).

7. Method according to any one of the preceding claims, **characterised in that** the chlorine compound of the disinfectant agent and/or of the disinfectant treatment medium is chlorine dioxide (ClO₂).

8. Method according to any one of the preceding claims, **characterised in that** the disinfectant agent and/or the disinfecting treatment medium are used in a gaseous and/or vaporous state or in a fluid state, for example in the form of an aqueous solution.

## Revendications

1. Procédé d'aseptisation de surfaces intérieures (2) de récipients par leur traitement avec un moyen de désinfection, dans lequel les surfaces intérieures (2) sont traitées avec un apport d'énergie germicide sous la forme de lumière UV et/ou sous la forme d'ultrason et/ou avec un fluide de traitement germicide qui contient un composé de chlore et qui est appliqué par des buses sur les surfaces intérieures (2), et le moyen de désinfection est du dioxyde de carbone (CO2) et est appliqué sous une forme liquide ou aqueuse et/ou sous une forme gazeuse et/ou de vapeur par des buses sur les surfaces intérieures (2), dans lequel par le traitement des surfaces intérieures (2) avec l'apport d'énergie germicide et/ou avec le fluide de traitement germicide la structure cellulaire, en particulier l'enveloppe extérieure ou la membrane de germes est interrompue, et dans lequel l'alimentation des surfaces intérieures (2) avec l'apport d'énergie germicide et/ou avec le fluide de traitement germicide est effectuée temporellement avant le traitement des surfaces intérieures (2) avec le moyen de désinfection.

2. Procédé selon la revendication 1, **caractérisé en ce que** le moyen de désinfection contient aussi le composé de chlore, et ce dans un dosage qui est inférieur au dosage du composé de chlore dans le fluide de traitement germicide.

3. Procédé selon la revendication 1 ou 2, **caractérisé en ce que** le fluide de traitement germicide est appliqué sous une forme liquide ou aqueuse et/ou sous une forme gazeuse et/ou de vapeur sur les surfaces intérieures (2).

4. Procédé selon l'une des revendications précédentes, **caractérisé en ce que** l'apport d'énergie est effectué par des éclairs de lumière par UV.

5. Procédé selon l'une des revendications précédentes, **caractérisé en ce que** le fluide de traitement germicide est un composé de chlore.

6. Procédé selon l'une des revendications précédentes, **caractérisé en ce que** le moyen de désinfection contenant du dioxyde de carbone (CO2) et le composé de chlore est mis à disposition à une pression se trouvant au-dessus de la pression ambiante et n'est relâché que lors d'une répartition sur les surfaces intérieures (2) à la pression ambiante.

7. Procédé selon l'une des revendications précédentes, **caractérisé en ce que** le composé de chlore du moyen de désinfection et/ou du fluide de traitement germicide est du dioxyde de chlore (ClO2).

8. Procédé selon l'une des revendications précédentes, **caractérisé en ce que** le moyen de désinfection et/ou le fluide de traitement germicide est utilisé dans un état gazeux et/ou sous forme de vapeur ou dans un état liquide, par exemple sous la forme d'une solution aqueuse.
